# EUROPEAN PATENT APPLICATION

(11) **EP 2 865 359 A1**
(43) Date of publication of application: **29.04.2015**
(21) Application number: 13189712.6
(22) Date of filing: 22.10.2013
(51) Int. Cl.: A61F 5/02

(54) **Back posture straightening device**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A back posture straightening device includes an cable having two ends secured together to form an endless loop, and a bridge engaged between two segments of the cable for separating the segments of the cable from each other and for forming an eight (8)-shaped structure for the back posture straightening device and for engaging onto shoulder portions of a user, the bridge includes a base and an bar engaged with the segments of the cable, and the base and the bar are adjustable relative to each other for adjusting a distance between the segments of the cable.

## Description

The invention relates to a back posture straightening device including comprising a cable including a first end and a second end secured together.

Typical elastic devices comprise an elastic cable for exercising muscle groups, but may not be used to exercise or straighten the shoulder portion or the back portion of the user.

The invention provides a back posture straightening device as defined in the claims.

The elastic cable can be supported with a retainer bridge to form an eight (8)-shaped structure.

In the drawing:
FIG. 1 is a rear plan view of a straightening device;
FIG. 2 is a front perspective view of the straightening device;
FIG. 3 is a partial exploded view of the straightening device;
FIG. 4 is a perspective view of the straightening device; and
FIGS. 5, 6 are front views of the straightening device.

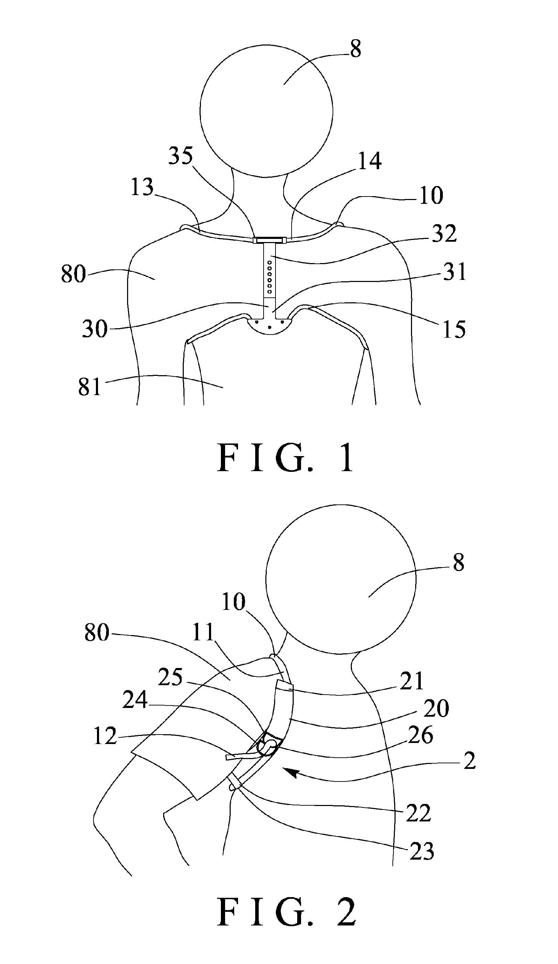

Referring to FIGS. 1-2, a back posture straightening device comprises an elastic cable 10 having two ends 11, 12 coupled together with a coupler 2 for forming an endless loop and having a middle portion 13 coupled with a retainer bridge 3 for forming an eight (8)-shaped structure and for engaging with the shoulder portion 80 of a user 8.

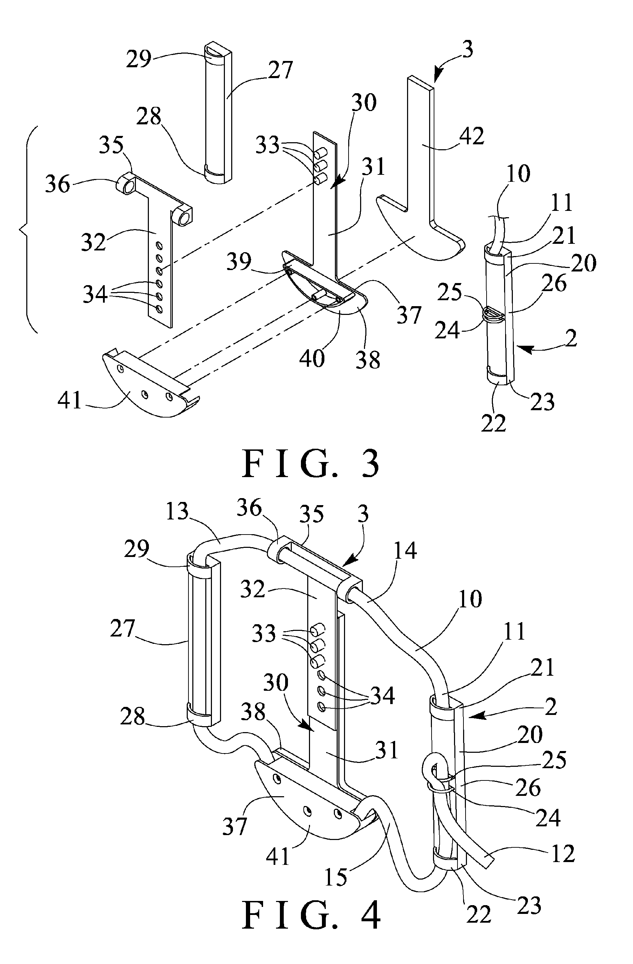

The coupler 2 includes a resilient pad 20 (FIGS. 3-6) having one end 21 secured to one end 11 of the cable 10, and having a loop 22 attached to the other end 23 for engaging with the other end 12 of the cable 10. The pad 20 includes one or more rings 24, 25 attached to the middle portion 26 of the pad 20 for securing the other end 12 of the cable 10 to the pad 20. The other end 12 of the cable 10 is engaged with the loop 22 and the rings 24, 25, and engaged with one of the rings 24 for locking to the other end 23 of the pad 20.

Another resilient pad 27 includes one or more loops 28, 29 for securing the middle portion 13 of the cable 10 to the pad 27, the middle portion 13 of the cable 10 may be spaced into two segments 14, 15 by the pads 20, 27.

The bridge 3 is engaged with the middle portion 13 of the cable 10 and located between the pads 20, 27 and/or the segments 14, 15 of the cable 10 for forming an eight (8)-shaped structure and for mounting onto the shoulder 80 and/or the back 81 of the user 8. The bridge 3 includes a beam 30 having a base 31 and a bar 32 secured together for spacing the segments 14, 15 of the cable 10 from each other. The base 31 of the beam 30 includes one or more (such as three) projections 33, and the bar 32 includes one or more (such as six) orifices 34 for engaging with the projections 33 and for adjusting the base 31 and the bar 32 relative to each other. The beam 30 includes a holder 35 provided on the bar 32 and having one or more (such as two) loops 36 for engaging with the upper segment 14 of the middle portion 13 of the cable 10.

The beam 30 includes an anchor 37 provided on the base 31 and having a plate 38 and a bulge 39 extended from the plate 38 for forming a channel 40 in the anchor 37 and/or between the plate 38 and the bulge 39 and for engaging with the lower segment 15 of the middle portion 13 of the cable 10, and a cover 41 attached to the plate 38 for retaining the second segment 15 of the cable 10 to the anchor 37 of the base 31. A panel 42 is attached to the base 31 and/or the plate 38 for reinforcing purposes.

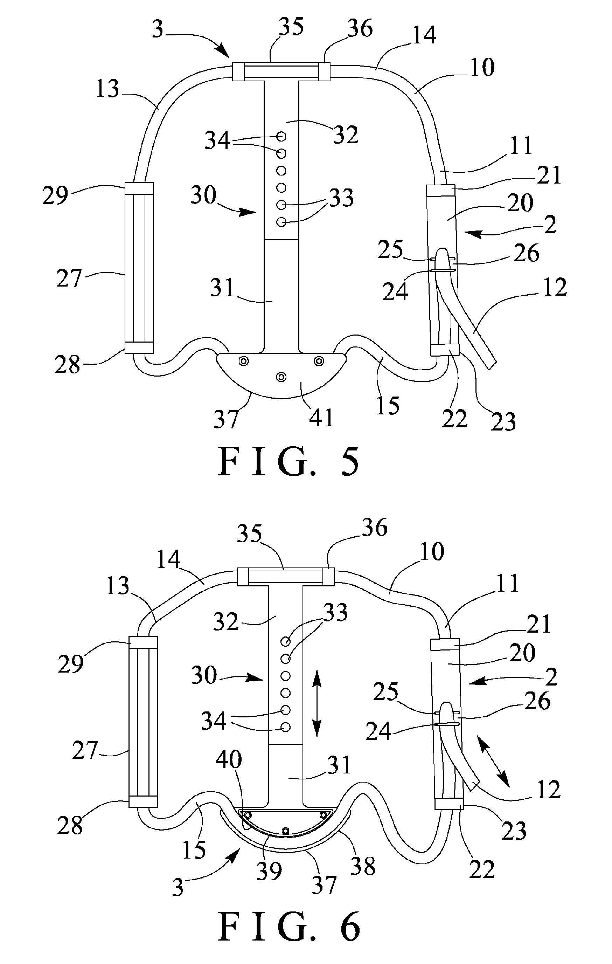

The eight (8)-shaped device may be engaged onto the shoulder portions 80 of the user 8 (FIGS. 1-2) for straightening the shoulder portion 80 and/or the back 81 of the user 8. The other end 12 of the cable 10 is adjustable relative to the pad 20 of the coupler 2 (FIGS. 2, 6).

## Claims

1. A back posture straightening device comprising a cable (10) including a first end and a second end (11, 12) secured together, **characterized in that**:
the cable (10) includes a middle portion (13) separated into a first segment and a second segment (14, 15), and
a bridge (3) is engaged between the first and the second segments (14, 15) of the cable (10), the bridge includes a base (31) and a bar (32) engaged with the first and the second segments (14, 15) of the cable (10) respectively and adjustable relative to each other.

2. The straightening device as claimed in claim 1, **characterised in that** the bridge (3) includes a holder (35) provided on the bar (32) for engaging with the first segment (14) of the cable (10).

3. The straightening device as claimed in claim 1 or 2, **characterised in that** the bridge (3) includes an anchor (37) provided on the base (31) for engaging with the second segment (15) of the cable (10).

4. The straightening device as claimed in claim 3, **characterised in that** the anchor (37) includes a plate (38) and a bulge (39) extended from the plate (389 for forming a channel in the anchor (37).

5. The straightening device as claimed in one of claims 1 to 4, **characterised in that** the base (31) includes at least one projection (33), and the bar (32) includes at least one orifice (34) for engaging with the projection (33) of the base (32).

6. The straightening device as claimed in one of claims 1 to 4, **characterised in that** the bar includes at least one projection, and the base includes at least one orifice for engaging with the projection of the bar.

7. The straightening device as claimed in one of claims 1 to 6 further comprising a coupler (2) secured to the first end (11) of the cable (10) and adjustable relative to the second end (12) of the cable (10).

8. The straightening device as claimed in one of claims 1 to 7 further comprising a pad (27) attached to the middle portion (13) of the cable (10).

9. The straightening device as claimed in one of claims 1 to 8, **characterised in that** the cable (10) is an elastic cable.
